(19) Europäisches Patentamt
European Patent Office
Office européen des brevets

(11) EP 0 577 040 B1

(12) **EUROPÄISCHE PATENTSCHRIFT**

(45) Veröffentlichungstag und Bekanntmachung des Hinweises auf die Patenterteilung:
**24.09.1997 Patentblatt 1997/39**

(51) Int Cl.[6]: **C07D 319/06**

(21) Anmeldenummer: **93110265.1**

(22) Anmeldetag: **28.06.1993**

(54) **Verfahren zur Herstellung von (3R,5S)6-Hydroxy-3,5-0-isopropyliden-3,5-dihydroxy-hexansäure-tert.-butylester**

Process for the preparation of (3R,5S)-6-hydroxy-3,5-O-isopropylidene-3,5-dihydroxy-hexanoic acid, tert.-butyl ester

Procédé de préparation de l'ester tert.-butyle de l'acide (3R,5S)6-hydroxy-3,5-O-isopropylidène-3,5-dihydroxy-hexanoique

(84) Benannte Vertragsstaaten:
**AT BE CH DE DK ES FR GB GR IE IT LI LU MC NL PT SE**

(30) Priorität: **02.07.1992 DE 4221658**

(43) Veröffentlichungstag der Anmeldung:
**05.01.1994 Patentblatt 1994/01**

(73) Patentinhaber: **HOECHST AKTIENGESELLSCHAFT**
**65926 Frankfurt am Main (DE)**

(72) Erfinder:
- **Beck, Gerhard, Dr.**
  **D-6000 Frankfurt am Main (DE)**
- **Jendralla, Joachim-Heiner, Dr.**
  **D-6230 Frankfurt am Main 80 (DE)**
- **Kesseler, Kurt, Dr.**
  **D-6237 Liederbach (DE)**

(56) Entgegenhaltungen:
**EP-A- 0 319 847** **DE-C- 4 128 345**

- **TETRAHEDRON LETTERS. Bd. 31, Nr. 18 , 1990 , OXFORD GB Seiten 2545 - 2548 G. WESS ET AL 'Stereoselective synthesis of HR780 anew highly potent hmg-coa reductase inhibitor'**
- **TETRAHEDRON LETTERS. Bd. 29, Nr. 13 , 1988 , OXFORD GB Seiten 1555 - 1556 M. KITAMURA ET AL 'A practical asymetric synthesis of carnitine'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 577 040 B1

**Beschreibung**

(3R,5S)6-Hydroxy-3,5-O-isopropyliden-3,5-dihydroxyhexansäure-tert.-butylester der Formel I

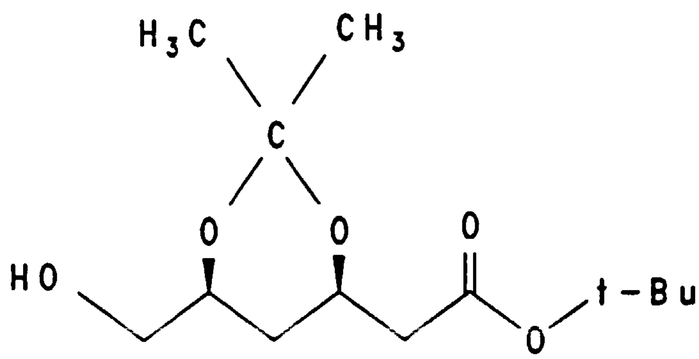

I

ist ein wertvoller Baustein zur Herstellung von HMG-CoA Reduktaseinhibitoren [EP-A-0319847 entspr. US-Patente Nr. 4,970,313 und Nr. 4,977,279; H. Jendralla et al., J. Med. Chem. 34, 2962 (1991) und dort zitierte frühere Arbeiten und deutsche Patentanmeldung P 4128345.7]. Letztere senken nach oraler Gabe den Plasmacholesterinspiegel beim Menschen (M.J.T.M. Mol et al. Lancet 1986, 936) und verringern damit das Risiko koronarer Herzerkrankungen [(LRC-CPPT J. Am. Med. Assoc. 251, 351 und 365 (1984)].

Ein Verfahren zur Herstellung der Verbindung der Formel I ausgehend von (4S,6S)-7-Benzyloxy-4,6-dihydroxyhept-1-en wird in der deutschen Patentanmeldung P 41 28 345.7 vorgeschlagen.

Die Herstellung der Verbindung der Formel I ausgehend von L-Apfelsäure wird in EP-A-0319847 beschrieben. Auf dem beschriebenen Weg sind HMG-CoA-Reduktase-Inhibitoren im Labormaßstab gut zugänglich [s. Vergleich der Synthesewege in J. Med. Chem. 34, 2962 (1991)]. In vergrößertem technischen Maßstab bereitet aber auch dieses Verfahren Probleme. Das Hauptproblem ist die nicht-kristalline Natur aller Zwischenstufen und des Synthons der Formel I, die mehrere chromatographische Reinigungen zwingend notwendig macht.

Es wurde nun gefunden, daß man die Verbindung der Formel I aus dem preiswerten ω-Chloracetessigsäureethylester bzw. unter Einsparung einer Synthesestufe aus dem ebenfalls kommerziell erhältlichen ω-Benzyloxy-acetessigsäureethylester vorteilhaft herstellen kann.

Die Erfindung betrifft daher ein Verfahren zur Herstellung der Verbindung der Formel I, dadurch gekennzeichnet, daß man ω-Benzyloxy-acetessigsäureethylester der Formel II

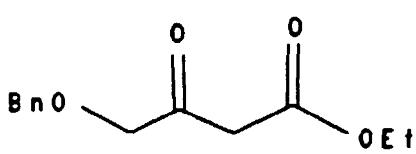

II

asymmetrisch hydriert zu 2(S)-Hydroxy-3-benzyloxy-buttersäureethylester der Formel III

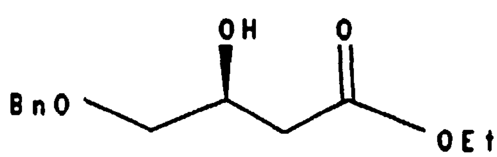

III

den β-Hxydroxyester der Formel III mittels Claisen-Kondensation in den β-Keto-δ-(S)-hydroxyester der Formel IV

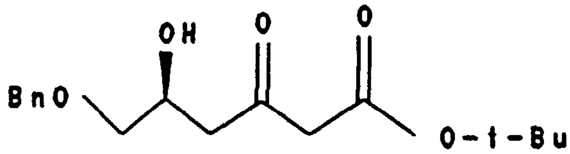

IV

überführt,
den erhaltenen Ester der Formel IV durch diastereoselektive Reduktion in 3(R),5(S)-Dihydroxy-6-benzyloxy-hexansäure-tert.-butylester der Formel V

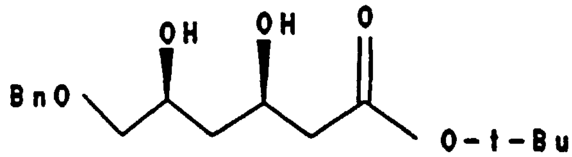

V

überführt,
in dem Dihydroxyester der Formel V unter Bildung des Acetonids der Formel VI

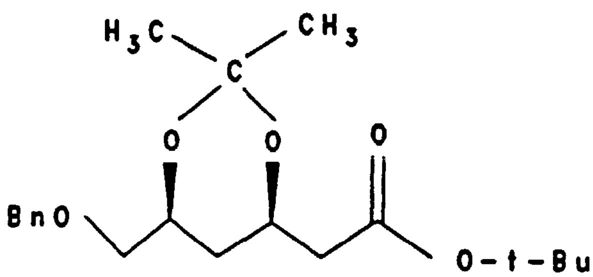

VI

die Hydroxygruppen schützt und aus dem Acetonid der Formel VI unter Bildung der Verbindung der Formel I die Benzylschutzgruppe entfernt.

Die Verbindung der Formel II ist kommerziell erhältlich oder kann aus ω-Chloracetessigsäureethylester in bekannter Weise erhalten werden. Das Prinzip des Verfahrens ist aus folgendem Schema ersichtlich:

## Schema:

### Synthese des Laktonbausteins der Formel I aus ω-Chloracetessigsäure-ethylester

ω-Chloracetessigsäure-ethylester

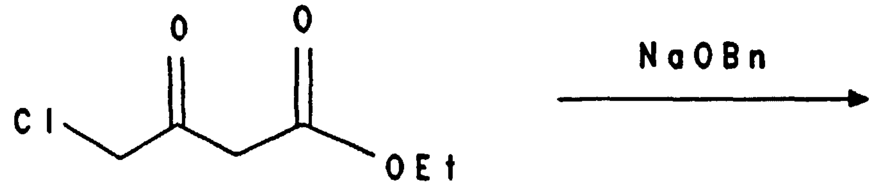


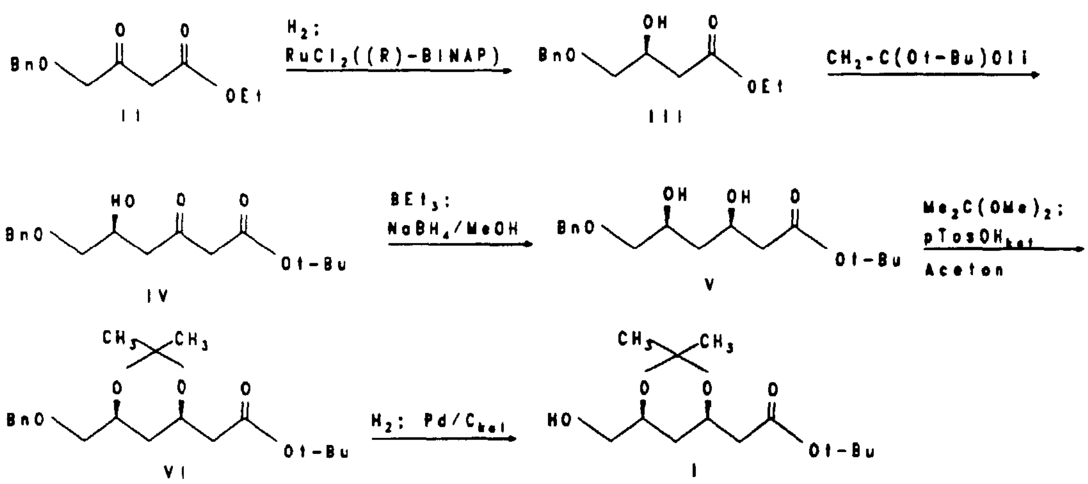


Das erfindungsgemäße Verfahren wird zweckmäßigerweise wie folgt durchgeführt. Der β-Ketoester II wird in bekannter Weise aus ω-Chloracetessigsäureethylester gewonnen und kann durch Vakuumdestillation am Dünnschichtverdampfer problemlos im Multikilogramm-Maßstab gereinigt werden. Asymmetrische Hydrierung von Verbindung II liefert 2(S)-Hydroxy-3-benzyloxy-buttersäureethylester der Formel III. Die asymmetrische Hydrierung wird bevorzugt nach dem Prinzip von Y. Noyori [M. Kitamura et al. Tetrahedron Lett. 29, 1555 (1988)] mit einem Ruthenium(II)-(R)-BINAP-Katalysator (BINAP ist 2,2'Bis-(diphenylphosphino)-1,1'binaphthyl) unter Anwendung einer vereinfachten in situ - Herstellung des Katalysators [M. Kitamura et al. Tetrahedron Lett. 32, 4163 (1991)] durchgeführt. Die asymmetrische Hydrierung am konkreten Substrat der Formel II ist zwar in der Literatur beschrieben (M.Kitamura et al., J.Am. Chem.Soc. 1988, 110, Seite 629-631), erfolgt dort aber mit relativ schlechter Enantioselektivität (78% ee) und einem relativ schlechten Substrat/Katalysator-Verhältnis (S/C 700/1) bei hohem Wasserstoffdruck (100 atm) mittels des Katalysators $RuBr_2$[(S)-binap], der gemäß Fußnote 9 (Seite 630) durch Behandlung von schwer Zugänglichem Ru $(OCOCH_3)_2$(BINAP) mit zwei Äquivalenten HBr hergestellt werden muß. Wir haben die Enantioselektivität (bis 98% ee), die Praktikabilität (Anwendung des einfach zugänglichen in situ-Katalysators (in situ-Ruthenium(II)-chlorid-(R)-BINAP-Katalysator), Anwendung eines wesentlich niedrigeren Wasserstoffdrucks (< 5 atm $H_2$)) und die Ökonomie dieser Reaktion (S:C wesentlich größer als 1000:1) deutlich verbessert. Sie wird bevorzugt in einem Autoklaven unter Verwendung eines polaren Lösungsmittels bei erhöhter Temperatur unter schwachem Wasserstoffdruck durchgeführt, besonders bevorzugt in Ethanol bei 100° C unter 4 atm Wasserstoff für 6 - 12 Stunden. In der besonders bevorzugten Arbeitsweise wird das Lösungsmittel anschließend im Vakuum entfernt und der Rückstand durch Vakuumdestillation am Dünnschichtverdampfer gereinigt. Im Multikilogramm-Maßstab kann diese Reaktion mit einem Substrat/Katalysator- Verhältnis bis ca. 2000: 1 durchgeführt werden. Die destillierte Verbindung der Formel III hat eine chemische

Reinheit > 98 % (GC) und eine optische Reinheit von 96 - 98 % es (HPLC-Analyse auf ®Chiralcel OD oder $^1$H-NMR-Analyse mit ®-Optishift). Die Ausbeute beträgt 90 - 97 % nach Destillation. Die Claisen-Kondensation von Verbindung III mit einem Überschuß des Enolats von Essigsäure-tert.-butylester ergibt den β-Keto-δ-(S)-hydroxy-Ester der Formel IV. Bevorzugt werden 4 Äquivalente des Enolats durch Reaktion mit 4 Äquivalenten LDA (LDA ist Lithiumdiisopropylamid) bei -40° C in THF erzeugt und dann bei Raumtemperatur mit Verbindung III zu Verbindung IV umgesetzt, die kristallin ist und im Multikilomaßstab durch Umkristallisation oder alternativ über ihren Lithiumbromid-Komplex (analog zu US 4,452,994) gereinigt werden kann. Die in 75 - 90 % Ausbeute erhaltene Verbindung der Formel IV hat eine optische Reinheit von 97,5 % ± 1 % ee (HPLC-Analyse auf Chiralcel OD). Auch der weitere Umsatz der rohen, ungereinigten Verbindung der Formel IV (siehe Beispiele) ergibt ein Endprodukt der Formel I von hoher Qualität.

Innerhalb der gesamten Synthesesequenz zum Baustein der Formel I müssen keine chromatographischen Reinigungen von Zwischenprodukten durchgeführt werden, da die Verbindungen der Formeln II und III leicht destillativ, die Verbindungen der Formeln IV und (oder) V leicht über Lithiumbromid-Komplexe gereinigt werden können.

Gereinigtes oder ungereinigtes Produkt der Formel IV kann mit sehr hoher Diastereoselektivität zum 3(R),5(S)-Dihydroxy-6-benzyloxy-hexansäure-tert.-butylester der Formel V reduziert werden. Bevorzugt wird diese Reduktion mit Natriumborhydrid/Triethylboran in Methanol bei niedriger Temperatur durchgeführt [analog K. Narasaka, F.-C. Pai Tetrahedron 40, 2233 (1984)]. Der Einsatz dieses Prinzips zur Generierung der korrekten Relativkonfiguration von HMG-CoA Reduktase Inhibitoren ist bekannt [EP-A-0319847; F. G. Kathawala et al. Helv. Chim. Acta 69, 803 (1986)] Die beiden Hydroxygruppen von der Verbindung V werden als Acetonid unter Bildung von Verbindung VI geschützt. Bevorzugt wird diese Reaktion mit Acetondimethylacetal (2,2-Dimethoxypropan) unter Katalyse von p-Toluolsulfonsäure durchgeführt (95 - 100 % Ausbeute). Die Benzylschutzgruppe von Verbindung VI kann mittels katalytischer Hydrierung entfernt werden (vgl. P 4128345.7), bevorzugt in Ethylacetet-Lösung mit Palladium auf Kohle unter ca. 10 bar Wasserstoff. Der fertige Baustein der Formel I kann (unter Verlusten) destillativ oder aber chromatographisch gereinigt werden. Die Verbindung I wird nach Reinigung in ca. 70 % Ausbeute erhalten (> 95 % ee und > 99 % chemische Reinheit). Die Overall-Ausbeute von Verbindung der Formel I aus kommerziell erhältlichem Ester der Formel II beträgt somit ca. 40 % über 5 Stufen.

Die Verwendung von Verbindung I zur Herstellung optisch reiner HMG-CoA Reduktase-Inhibitoren ist beschrieben in EP-A-0319847.

Die folgenden Beispiele entsprechen einer bevorzugten Ausführungsform des neuen Verfahrens. Sie sind bezüglich verwendeter Reagenzien, Katalysatoren, Lösungsmittel, Reaktionstemperaturen, Drücke, Reaktionszeiten, Aufarbeitungs-, Reinigungs- und Analysemethoden nicht limitierend für das Verfahren.

Beispiel 1

ω-Benzyloxy-acetessigsäureethylester II

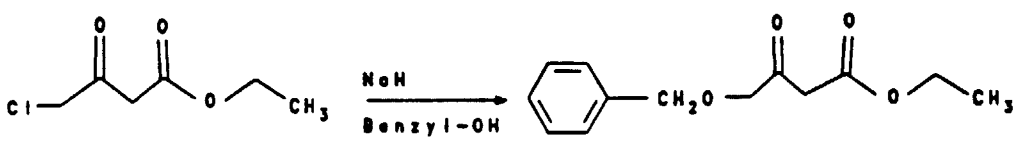


II

7,35 kg NaH Dispersion 55 - 60 %ig werden in 101,3 l Toluol suspendiert. Bei 20 - 25° C werden innerhalb einer Stunde 16,46 l Benzylalkohol zugetropft. Die Reaktion ist leicht exotherm (leichte Solekühlung). Man rührt noch etwa 2 Stunden nach, dann ist von der Wasserstoffentwicklung nichts mehr zu sehen. Ebenfalls bei 20 - 25° C tropft man innerhalb 1,5 Stunden 12,5 kg Chloracetessigester zu (leicht exotherm, Solekühlung). Danach wird noch 2 Stunden bei RT nachgerührt. GC Kontrole zeigt vollständige Umsetzung des Chloracetessigesters an. Der Ansatz wird mit 75,50 l 2n Citronensäure (9,64 kg Citronensäure in 67,75 l Wasser) leicht sauer gestellt (etwa pH 4), mit 10,00 l Toluol versetzt und ausgerührt. Die wäßrige Phase wird noch einmal mit 10,00 l Toluol ausgerührt. Die vereinigten org. Phasen werden mit Natriumsulfat getrocknet und einrotiert. Das resultierende Öl wird zweimal mit je 2,5 l n-Heptan verrührt (um das Weißöl des NaH zu entfernen). Das Öl wird abgetrennt. Die vereinigten Heptan-Gemische werden im Ausrührgefäß über Nacht stehen gelassen, da sich noch etwas Öl abscheidet. Am nächsten Morgen wird das Öl abgetrennt. Das gesamte Öl wird an einem zweistufigen Dünnschichtverdampfer bei 0,5 mbar destilliert, 1. Manteltemperatur 130° C, 2. Manteltemperatur 170° C.

Ausbeute an Verbindung II: 11,83 kg = 65,6 %, Sdp. ~ 170° C/0,5 Torr. Analyse: 25 m "fused silica" OV1 -Kapillarsäule, Injektor 250° C, Detektor 280° C, Säulentemp. 100° C für 2 Min., mit 30° C/Min. auf 140° C, dort 6,5 Min. $t_{ret}$: Benzylalkohol 1,2 Min., Chloracetessigester 2,0 Min., Verbindung der Formel II 7.1 Min.

Beispiel 2

2(S)-Hydroxy-3-benzyloxy-buttersäureethylester III

Asymmetrische Hydrierung unter Verwendung eines festen in-situ-Katalysators

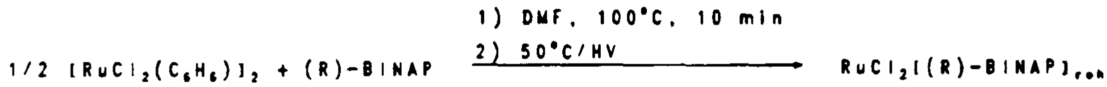


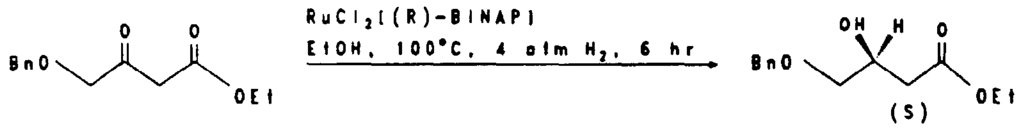


II III

Prinzip: M. Kitamura et. al. Tetrahedron Lett. 29, 1555 (1988); vereinfachte Katalysator-Herstellung, M. Kitamura et. al. Tetrahedron Lett. 32, 4163 (1991).

a) Herstellung des Katalysators

Ein 10 ml Kölbchen wurde mit 43,5 mg Benzol-ruthenium (II)-chlorid dimer und 113,7 mg (R)-BINAP befüllt. 3 ml DMF, durch das 5 min Argon geperlt worden war, wurden zugegossen, nachdem der Kolben mit Argon gefüllt worden war. Die resultierende Lösung (gemäß Kitamura et. al. Suspension) wurde unter Argon 10 min auf 100° C erhitzt (vorgeheiztes Ölbad). Die dunkelrote Lösung wurde abgekühlt und bei Pumpenvak./50 - 70° C unter heftigem Rühren konzentriert (~ 5 min), der zurückbleibende Feststoff wurde noch 30 Min. im vollen HV getrocknet. Er wurde auf RT abgekühlt, mit Argon belüftet und unter Argon mit einem Spatel von der Wand abgeschabt.

b) Herstellung von Verbindung III

In einem 250 ml-Schüttelautoklaven-Glaseinsatz war die Lösung von 41,1 g Ketoester (aus Beispiel 1) in 40,0 ml abs. Ethanol 2 Std. mit Argon durchperlt worden. 85,0 mg (107 µmol ber. als $RuCl_2$[(R)-BINAP]) des obigen Katalysators wurden zugegeben. Der Glaseinsatz wurde mit einem Argon-gefüllten Ballon verschlossen. Der Katalysator ging beim Umschwenken vollständig in Lösung. Der Einsatz wurde in einen $N_2$-gefüllten Autoklaven eingesetzt. Reste Luft, $N_2$ und Argon wurden durch dreimaliges Aufdrücken von 50 atm $H_2$ und langsames Entspannen beseitigt. Dann wurden 2 atm $H_2$ aufgedrückt. Es wurde auf 100° C geheizt. Dann wurde der $H_2$-Druck exakt auf 4 atm gestellt. Es wurde 6 Std. bei 100° C geschüttelt. Über Nacht ließ man auf RT abkühlen.

Es wurde mit $N_2$ gespült, der Autoklav geöffnet. Die Lösung wurde einrotiert. Man erhielt ein gelbes Öl.
GC zeigte quantitative Umwandlung des AM (Ausgangsmaterial) ins Produkt an (GC-Reinh. Rohprodukt 98,5 %).
Das Rohprodukt wurde über eine 3/8" cm Vigreux-Kolonne im HV fraktioniert:

Frkt. 1 Sdp. 80 - 105° C/ 0.01 Torr; Bad 140-150° C;
1,00 g farbl. Öl
Frkt. 2 Sdp. 107 - 112° C/ " " 150° C;
8,55 g farbl. Öl

| | | | |
|---|---|---|---|
| Frkt. 3 | Sdp. 114 - 116° C/ " | " | 150° C; |
| 29,20 g farbl. Öl | | | |
| Frkt. 4 | Sdp. 116 - 118° C " | " | 150-180° C |
| 0,62 g farbl. Öl | | | |
| ======== | | | |
| 39,37 g farbl. Öl | | | |

Gesamtausbeute: 39,37 g (165 mml) = 95 % d. Th.

| GC-Analyse: | | | |
|---|---|---|---|
| Frkt. 1: | Produktreinheit | 77,0 % | ~ 16.0 % AM → verworfen |
| Frkt. 2: | " | 95,2 % | ~ 1,4 % AM |
| Frkt. 3: | " | 99,7 % | ~ 0 % AM |
| Frkt. 4: | " | 94,3 % | - |

Ausbeute der sauberen Frkt. (2 - 4): 38,37 g (161 mmol) = 92,5 % d. Th.

Drehwert von Frkt. 2 + 3:

59,0 mg/5 ml EtOH abs. unvergällt, d. h. c = 1,18;
gemessen: 20° C, D-Linie: -0,105

$$[\alpha]_D^{20} = \frac{-0,105 \bullet 100}{1,18} = -8,90°$$

Optische Reinheit HPLC: Frkt. 3: 98 % ee

Optische Reinheit (Optishift): Frkt. 2: > 95 % ee

Beispiel 3

2(S)-Hydroxy-3-benzyloxy-buttersäureethylester III

Asymmetrische Hydrierung unter Verwendung eines in-situ Katalysators in DMF-Lösung

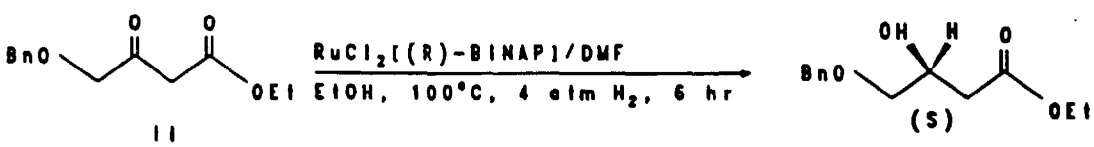


II

III

Katalysatorherstellung, Durchführung der Hydrierung erfolgte gemäß Beispiel 2 mit dem Unterschied, daß die DMF-Lösung des Katalysators nicht zur Trockne eingeengt wurde, sondern als Lösung der ethanolischen Substratlösung unter Argon zugesetzt wurde.

Das Substrat/Katalysator-Verhältnis betrug 1000 : 1.

Hydriert wurde bei 100° C/4 atm $H_2$-Druck / 6 Std.

Das Rohprodukt wurde im Hochvakuum fraktioniert:

| | | |
|---|---|---|
| Frkt. 1 | 90-110° C/~ 0,01 Torr 0,83 g farbl. Öl | verworfen |

(fortgesetzt)

| | | |
|---|---|---|
| Frkt. 2 | 111-120° C/~ 0,01 Torr 11,84 g farbl. Öl | Reinh. (GC): 98,0 % |
| Frkt. 3 | 123-125° C/~ 0,01 Torr 27,74 g farbl. Öl | Reinh. (GC): 97,9 % |

Ausbeute der sauberen Frkt. (2 + 3): 39,58 g (166 mmol) = 95,4 % d. Th;
Reinheit: 98 %
Drehwert von Frkt. 2 + 3: 58,9 mg/5 ml EtOH abs. unvergällt, d. h. c = 1,178;
gemessen: 20° C, D-Linie: - 0,107

$$[\alpha]_D^{20} = \frac{-0,107 \bullet 100}{1,178} = -9,083°$$

Optische Reinheit (Optishift): Frkt. 2: > 95 % ee.

Beispiel 4

2(S)-Hydroxy-3-benzyloxy-buttersäureethylester III.

Asymmetrische Hydrierung unter Verwendung eines in-situ Katalysators in DMF-Lösung

| Materialien: | |
|---|---|
| 10 kg | Ketoester II |
| 18.0 g | (R)-(+)-BINAP |
| 7.0 g | Benzolruthenium(II)chlorid dimer |
| 10 l | Ethanol abs., mit Toluol vergällt |
| 900 ml | N,N-Dimethylformamid |

2.0 l N,N-Dimethylformamid werden frisch im Hochvakuum von 4A-Molekularsieben abdestilliert, wobei eine Mittelfraktion (Sdp. 35-40°C) gesammelt wird. In einem Zweiliterkolben mit Rührmagnet, Argon-Zuleitung, Blasenzähler werden 900 ml frisch destilliertes N,N-Dimethylformamid vorgelegt und Argon durchgeperlt. 18.0 g (R)-(+)-BINAP und 7.0 g Benzolruthenium(II)chlorid dimer werden zugegeben. Die resultierende Lösung wird 15 Minuten mit Argon durchgeperlt und dann 10 Minuten in ein auf 100°C vorgeheiztes Ölbad tief eingetaucht, wobei man das Argon weiter durch die Lösung perlen läßt. Die entstehende klare, dunkelrotbraune Lösung läßt man unter Argon auf Raumtemperatur abkühlen. In der Zwischenzeit wird ein 30 l Stahlautoklav mit 10 l abs. Ethanol (mit Toluol vergällt) und 10 kg Ketoester II (aus Beispiel 1) befüllt. Durch die Lösung wird ein kräftiger Argonstrom geperlt. Die Katalysatorlösung wird mittels einer Doppelkanüle mit Argon zu der Substratlösung übergedrückt.
Der Autoklav wird verschlossen, dann mit Wasserstoff gespült. Es wird 2 bar Wasserstoff aufgedrückt, dann auf 100°C geheizt. Nachdem diese Temperatur erreicht ist, stellt man den Wasserstoffdruck auf exakt 4 bar ein und rührt 12 Stunden bei diesen Bedingungen. Man läßt abkühlen. Eine entnommene Probe zeigt bei GC-Analyse > 99% Hydrierung an. HPLC-Analyse auf $^R$Chiralcel OD zeigt 97.4% ee des Rohprodukts an. Der Autoklav wird mit Stickstoff gespült, der Inhalt wird entnommen und das Ethanol im Vakuum entfernt. Anschließend wird das DMF bei 60°C Badtemperatur im Hochvakuum entfernt. Der Rückstand wird an einem Dünnschichtverdampfer (140-150°C Manteltemperatur, 0,1 Torr) destilliert. Man erhält 9,70 kg (96,2% d.Th.) eines blaßgelben (fast farblosen) Öls, GC-Reinheit: > 98%, optische Reinheit ($^R$Chiralcel OD) : 97,1%, $[\alpha]D^{20}$ = - 8.3° (c = 1.2, in Ethanol abs.).
Aus dem Destillationssumpf (schwarzes zähes Öl) kann (R)-(+)-BINAP zurückgewonnen werden.

Beispiel 5

3-Oxo-5(S)-hydroxy-6-benzyloxy-hexansäure-tert.-butylester IV

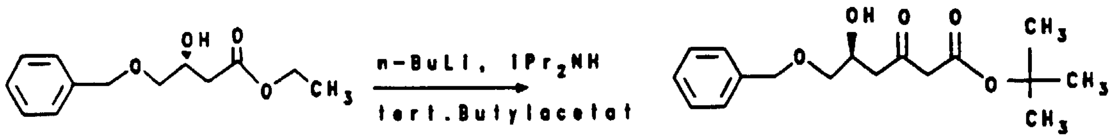


III IV

Materialien:

9,4 l (66 Mol) Diisopropylamin
13,4 l THF (abs. nach Karl Fischer)
43,6 l = 29,65 kg (69,76 Mol) n-BuLi (in Hexan 15%ig)
9,0 l (67,6 Mol) tert. Butylacetat
4 kg (6,30 Mol) Verbindung III (aus Beispiel 2, 3 oder 4)
32 l Toluol

9,4 l Diisopropylamin werden in 9,4 l THF abs. gelost und bei 0°C werden 29,65 kg n-BuLi zugetropft. Dann wird 30 Minuten bei Raumtemperatur nachgerührt und auf -40°C gekühlt. Bei dieser Temperatur werden 9 l tert. Butylacetat zugetropft. Es wird 1 Stunde bei -40°C nachgerührt. Dann werden 4 kg Verbindung III, gelöst in 4 l THF, zugetropft. Der Ansatz wird 2 Stunden bei -40°C nachgerührt. Danach wird ohne weitere Kühlung mit 7,4 l Wasser versetzt, 10 Minuten nachgerührt. Anschließend wird 2 x mit je 16 l Toluol extrahiert. Die vereinigten Toluol-Hexan-Phasen werden getrocknet und einrotiert. Das Produkt wird am Rotationsverdampfer halbfest. Ausbeute: 8.0 kg = 155,7% an Verbindung IV.

Beispiel 6

Option der Reinigung des Ketoesters IV aus Beispiel 5 über einen Lithiumbromid-Komplex

62 g des Rohprodukts aus Beispiel 5 werden in 260 ml Petrolether (40-80°C) gelöst (falls nötig, dann Toluol zutropfen, bis eine klare Lösung entsteht). 55 g wasserfreies Lithiumbromid werden zugegeben, und die Suspension wird dann eine Stunde unter Feuchtigkeitsausschluß gerührt. Man saugt den Lithiumbromid-Komplex ab und wäscht ihn mit 50 ml Petrolether. Man bringt die Kristalle in 100 ml Wasser und 250 ml Methyl-tert.-butyl-ether ein und rührt 30 Minuten heftig. Man trennt die organische Phase ab, entfernt das Lösungsmittel im Vakuum und nimmt den öligen Rückstand in 100 ml Diisopropylether auf. Dabei tritt Kristallisation ein. Der Feststoff wird abgesaugt, mit 20 ml Diisopropylether gewaschen und im Vakuum getrocknet. Man erhält 29 g (82% d.Th. bezogen auf β-Hydroxyester III) farblosen Feststoff IV, der im Gegensatz zum Rohprodukt fast vollständig in der Enolform vorliegt ($^1$H-NMR), Schmelzpunkt 136°C.

Beispiel 7

3(R),5(S)-Dihydroxy-6-benzyloxy-hexansäure-tert.butylester V

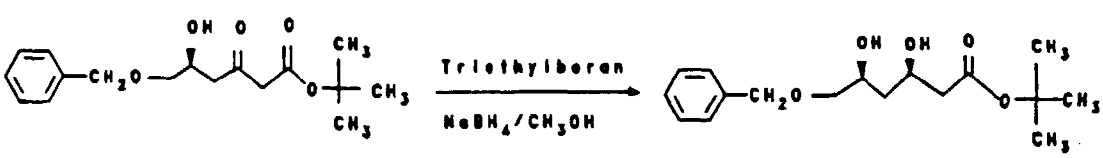


IV V

Materialien:

2,77 kg (100% = 1,94 kg = 6,29 Mol) Verbindung IV (Beispiel 5)
24,5 l THF
13,3 l Triethylboran 1 molar in Hexan

680,9 g Natriumborhydrid
17,5 l Methanol
13,75 l Wasserstoffperoxid

2,77 kg Verbindung IV werden bei 20-25°C in 24,5 l THF gelöst. Man läßt relativ schnell bei gleicher Temperatur 13,3 l Triethylboran zulaufen. Die Reaktion ist endotherm. Es wird 10 Minuten bei 20-25°C nachgerührt und danach auf -60°C gekühlt. Bei dieser Temperatur werden 680,9 g Natriumborhydrid zugegeben. Auch diese Zugabe ist ohne exotherme Reaktion. Sofort danach wird mit dem Eintropfen von 11,5 l Methanol begonnen (Dauer etwa 1,5 - 2 Stunden, exotherme Reaktion bis max. -60°C). Nach Beendigung der Zugabe wird 2 Stunden bei -60°C nachgerührt. Nach etwa 1,5 Stunden (DC Kontrolle) wird der Ansatz aus dem Kessel genommen und in die Zulaufgefäße gefüllt. 13,75 l Wasserstoffperoxid 35%ig werden mit 13,75 l Wasser und 6 l Methanol in den kalten Kessel gegeben und unter Rühren und Kühlung bei anfänglich 0°C bis max 15°C wird der Ansatz aus den Zulaufgefäßen zudosiert bei voller Trockeneiskühlung (Dauer etwa 1 Stunde). Nach beendeter Zugabe wird der Ansatz mit THF behandelt. Die wäßrige Phase wird abgetrennt und noch zweimal mit je 13 l Toluol und einmal mit 13 l Essigester ausgerührt. Die erhaltene organische Phase wird einrotiert.
Ausbeute: 2,20 kg = 122,6% hellgelbes Öl (Verbindung V).

Beispiel 8

(3R,5S)-6-Benzyloxy-3,5-O-isopropyliden-3,5-dihydroxy-hexansäure-tert.-butylester VI

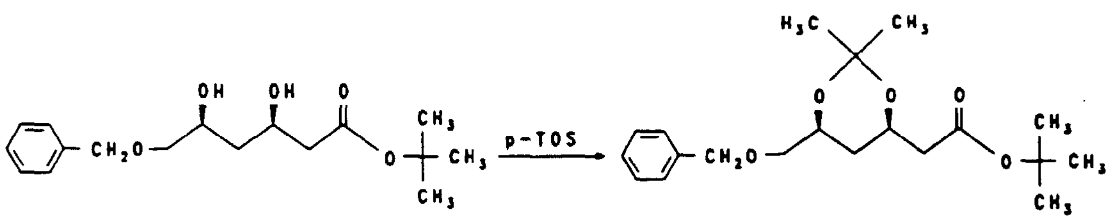


V VI

Materialien:

2,2 kg (7,087 Mol) "Diol" (Verbindung V aus Beispiel 7)
30 l Aceton
1,506 kg (14,17 Mol) 2,2-Dimethoxypropan
0,135 kg (0,708 Mol) p-Toluolsulfonsäure
5 l Essigester

2,2 kg Verbindung V werden bei Raumtemperatur in 30 l Aceton gelöst. Bei Temperaturen von 20 bis 25°C gibt man 1,506 kg 2,2-Dimethoxypropan 98%ig (= 1,778 l) und anschließend 0,135 kg p-Toluolsulfonsäure-Hydrat zu und rührt 3 Stunden bei diesen Temperaturen nach. Die ursprüngliche farblose Lösung färbt sich nach 1 - 2 Stunden langsam gelblich. Der Ansatz bleibt über Nacht bei 25°C stehen. Am nächsten Morgen engt man im Vakuum ein und löst den Rückstand (Öl) in 5 l Essigester. Die organische Phase wird nun einmal mit 2,5 l ges. $NaHCO_3$-Lösung und einmal mit 2,5 l ges. NaCl-Lösung gewaschen. Man trocknet über $Na_2SO_4$, klärt und engt im Vakuum ein. Rohausbeute: 2,38 kg (96% der Theorie) Öl.

Beispiel 9

(3R,5S)-6-Hydroxy-3,5-O-isopropyliden-3,5-dihydroxy-hexansäure-tert.-butylester (I)

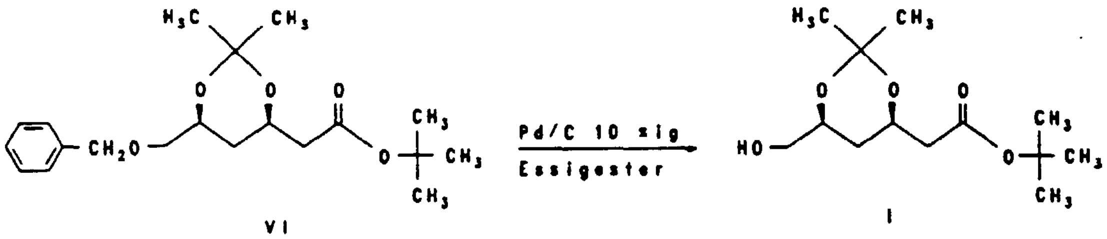


VI I

Materialien:

2,38 kg (6,79 Mol) Verbindung VI aus Beispiel 8
30 l Essigester
0,238 kg Pd /C 10%ig

2,38 kg Verbindung VI werden in 30 l Essigester gelöst. Unter $N_2$ setzt man 0,238 kg Pd/C 10%ig zu und hydriert bei 10 bar $H_2$ im Rührautoklaven 8 Stunden lang. Am nächsten Morgen wird die Reaktion mittels DC kontrolliert. Wenn die Umsetzung noch nicht fertig ist, wird abgesaugt, neuer Katalysator zuzugeben und weiter hydriert. Wenn die Hydrierung fertig ist, wird vom Katalysator abgesaugt und einrotiert. Rohausbeute: 1,55 kg (87,9% der Therorie, Öl). Das resultierende Öl wird in zwei Chromatographie-Säulen gereinigt. Dabei werden 8 kg rohes Öl mit 8 l MTB-Ether(Methyl-ter.-butyl-ether)/Cyclohexan 1:1 auf eine mit MTB-Ether/Cyclohexan 1:1 geflutete 15 kg Kieselgelsäule aufgezogen und mit gleichem Fließgemisch eluiert. Der Vorlauf von 50 l wird verworfen. Danach werden 20 Fraktionen à 5 l abgenommen und mittels DC kontrolliert. Die Produktfraktionen werden einrotiert.
Ausbeute: 600 g Verbindung I (Ab der Esterkondensation d.h. ab Beispiel 5 ergibt sich damit eine Ausbeute von 43,6% über 4 Stufen).

**Patentansprüche**

1. Verfahren zur Herstellung von (3R,5S)6-Hydroxy-3,5-O-isopropyliden-3,5-dihydroxy-hexansäure-tert.-butylester der Formel I

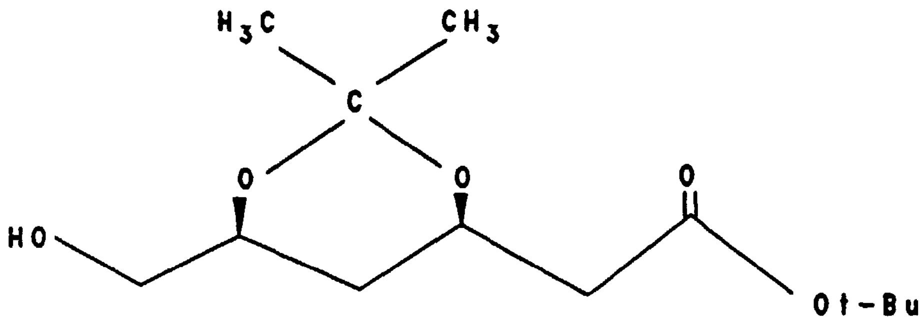

I

dadurch gekennzeichnet, daß man ώ-Benzyloxy-acetessigsäureethylester der Formel II

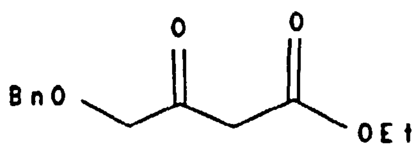

II

asymmetrisch hydriert zu 2(S)-Hydroxy-3-benzyloxy-buttersäureethylester der Formel III

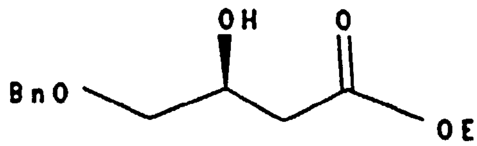

III

den β-Hydroxyester der Formel III mittels Claisen-Kondensation in den β-Keto-δ-(S)-hydroxyester der Formel IV

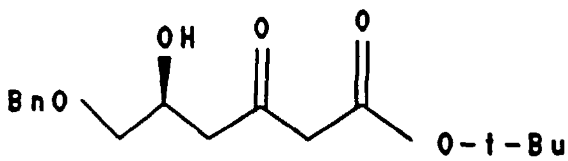

IV

überführt,
den erhaltenen Ester der Formel IV durch diastereoselektive Reduktion in 3(R),5(S)-Dihydroxy-6-benzyloxy-hexansäure-tert.-butylester der Formel V

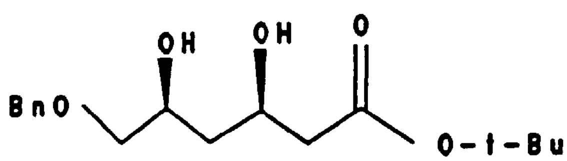

V

überführt,
in dem Dihydroxyester der Formel V unter Bildung des Acetonids der Formel VI

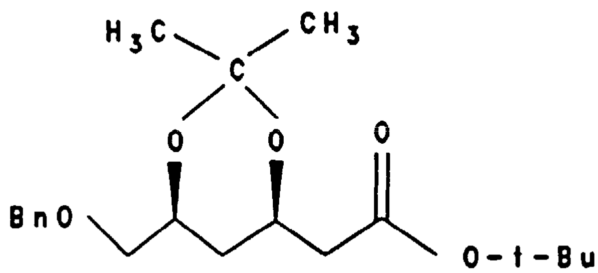

VI

die Hydroxygruppen schützt und aus dem Acetonid der Formel VI unter Bildung der Verbindung der Formel I die Benzylschutzgruppe entfernt.

**2.** Verfahren gemäß Anspruch 1, dadurch gekennzeichnet, daß die asymmetrische Hydrierung von Verbindung II zu Verbindung III bei einem hohen Substrat/Katalysator-Verhältnis unter Verwendung eines in situ - Ruthenium(II) chlorid-(R)-BINAP-Katalysators durchgeführt wird.

**3.** Verfahren gemäß Anspruch 1, dadurch gekennzeichnet, daß die asymmetrische Hydrierung von Verbindung II zu Vebrindung III bei

a) einem hohen Substrat/Katalysator-Verhältnis (> > 1000 : 1),
b) unter Verwendung eines sehr leicht zugänglichen in situ - Ruthenium(II)-chlorid-(R)-BINAP-Katalysators und
c) einem niedrigen, technisch einfach zu handhabendem Wasserstoffdruck (< 5 atm $H_2$)
durchgeführt wird.

4. Verfahren gemäß Anspruch 1, dadurch gekennzeichnet, daß man die Verbindung I in hoher chemischer und optischer Reinheit erhält ohne Zwischenstufen chromatographieren zu müssen.

## Claims

1. A process for preparing tert-butyl (3R,5S)-6-hydroxy-3,5-O-isopropylidene-3,5-dihydroxyhexanoate of the formula I,

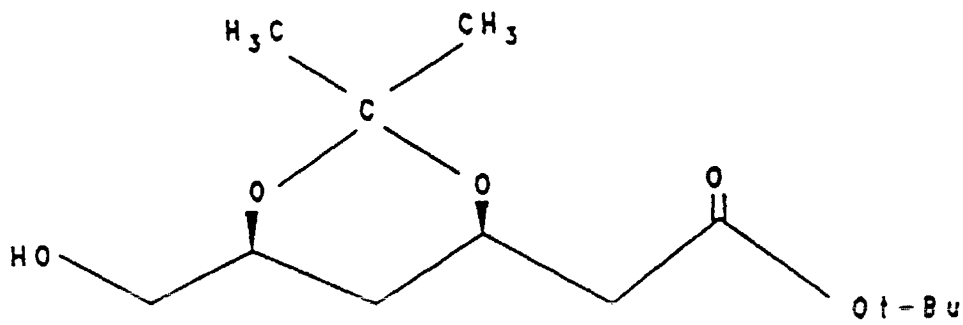

I

which comprises asymmetrically hydrogenating ethyl ω-benzyloxyacetoacetate of the formula II

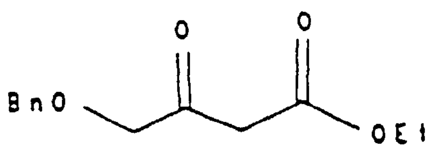

II

to give ethyl 2(S)-hydroxy-3-benzyloxybutyrate of the formula III,

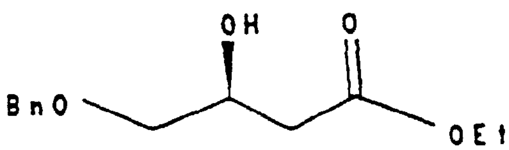

. III

converting the β-hydroxy ester of the formula III by means of Claisen condensation into the β-keto-δ-(S)-hydroxy ester of the formula IV,

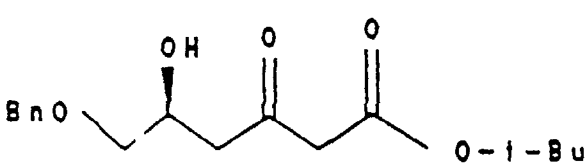

IV

converting the resultant ester of the formula IV by diastereoselective reduction into tert-butyl 3(R),5(S)-dihydroxy-6-benzyloxyhexanoate of the formula V,

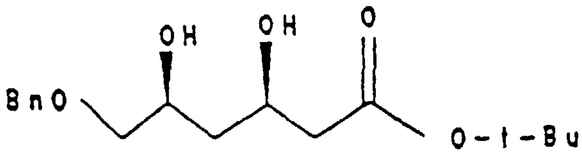

V

protecting the hydroxyl groups in the dihydroxy ester of the formula V, with the formation of the acetonide of the formula VI,

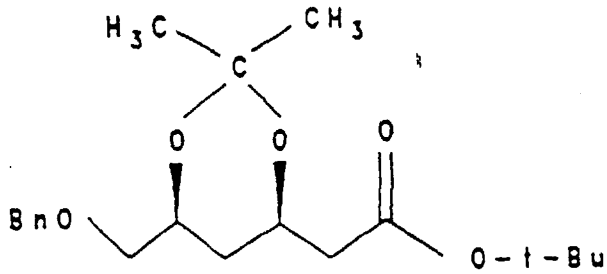

V I

and removing the benzyl protective group from the acetonide of the formula VI, with the formation of the compound of the formula I.

2. The process as claimed in claim 1, wherein the asymmetric hydrogenation of compound II to give compound III is carried out at a high substrate/catalyst ratio using an in-situ ruthenium(II) chloride-(R)-BINAP catalyst.

3. The process as claimed in claim 1, wherein the assymetric hydrogenation of compound II to give compound III is carried out.

   a) at a high substrate/catalyst ratio (>>1000:1),
   b) using an in-situ ruthenium(II) chloride-(R)-BINAP catalyst which is very readily accessible and
   c) under a low hydrogen pressure (<5 atm $H_2$) which is simple to implement from an industrial point of view.

4. The process as claimed in claim 1, wherein the compound I is obtained at high chemical and optical purity without having to subject intermediate stages to chromatography.

**Revendications**

1. Procédé pour la préparation du (3R,5S)-6-hydroxy-3,5-O-isopropylidène-3,5-dihydroxyhexanoate de tert-butyle de formule I,

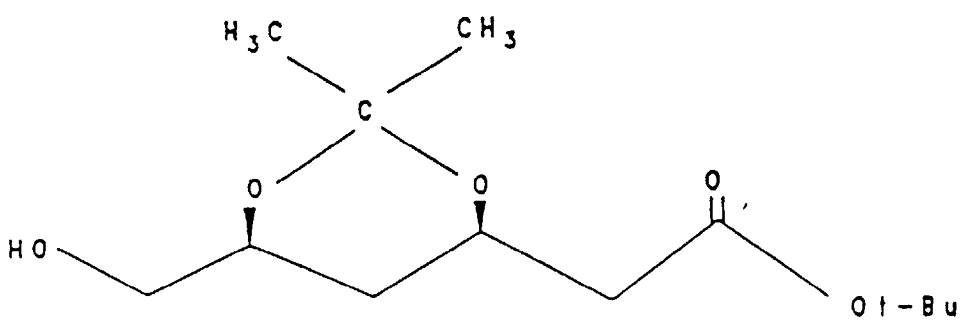

I

caractérisé en ce que l'on soumet à une hydrogénation asymétrique l'ω-benzyloxyacétoacétate d'éthyle de formule II

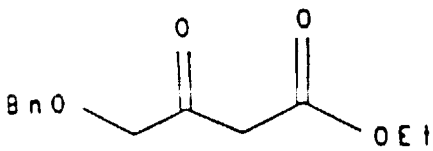

II

pour aboutir au 2(S)-hydroxy-3-benzyloxybutyrate d'éthyle de formule III

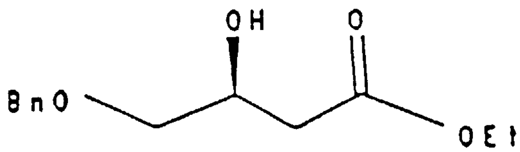

III

on convertit le β-hydroxyester de formule III, par une condensation de Claisen, en le β-céto-δ-(S)-hydroxyester de formule IV

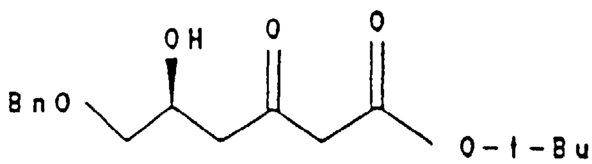

IV

on convertit l'ester de formule IV obtenu, par réduction diastéréosélective, en 3(R),5(S)-dihydroxy-6-benzyloxyhexanoate de tert-butyle de formule V

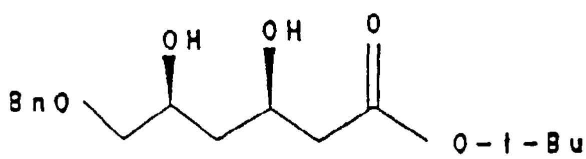

V

on protège les groupes hydroxy dans l'ester dihydroxylé de formule V, avec formation de l'acétonide de formule VI

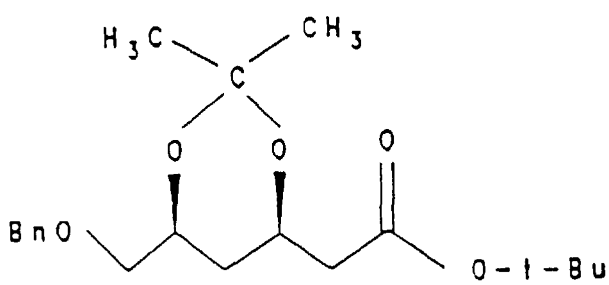

VI

et, on élimine le groupe protecteur benzyle de l'acétonide de formule VI, avec formation du composé de formule I.

2. Procédé selon la revendication 1, caractérisé en ce que l'hydrogénation asymétrique du composé II en composé III est effectuée à un rapport subtrat/catalyseur élevé, avec utilisation d'un catalyseur consistant en chlorure de ruthénium-(II)-(R)-BINAP in situ.

3. Procédé selon la revendication 1, caractérisé en ce que l'hydrogénation asymétrique du composé II en composé III est effectuée

a) avec un rapport substrat/catalyseur élevé(»1000:1),
b) avec utilisation d'un catalyseur consistant en chlorure de ruthénium-(II)-(R)-BINAP aisément accessible in situ et
c) sous une faible pression d'hydrogène (<5 atmosphères de $H_2$) aisée à manipuler industriellement.

**4.** Procédé selon la revendication 1, caractérisé en ce que l'on obtient le composé I en grande pureté chimique et optique, sans qu'il soit nécessaire de chromatographier des produits intermédiaires.